# EUROPEAN PATENT SPECIFICATION

(11) **EP 3 021 778 B1**
(45) Date of publication and mention of the grant of the patent: **05.09.2018**
(21) Application number: 14826819.6
(22) Date of filing: 17.07.2014
(51) Int. Cl.: A61B 18/22, A61B 18/04

(54) **VESSEL SEALING AND CUTTING DEVICES**
GEFÄSSVERSIEGELUNGS- UND -SCHNEIDVORRICHTUNGEN
DISPOSITIFS D'OBTURATION ET DE DÉCOUPE D'UN VAISSEAU

(30) Priority: 17.07.2013 US 201361847090 P
(43) Date of publication of application: 25.05.2016
(73) Proprietor: Asymmetric Medical Ltd., 7685400 Kfar Mordechai (IL)
(72) Inventor: ESHKOL, Moshe, 6091700 Harutzim (IL); WEISBERG, Ori, 7685500 Shdema (IL)
(74) Representative: Pearl Cohen Zedek Latzer Baratz UK LLP
(86) International application number: PCT/IL2014/050646
(87) International publication number: WO 2015/008286

(56) References cited:
- WO-A1-2013/084227
- US-A- 5 336 221
- US-A- 5 336 221
- US-A1- 2002 151 879
- US-A1- 2009 299 353
- US-A1- 2009 299 353
- US-A1- 2011 288 369
- US-A1- 2012 022 525
- US-A1- 2012 296 324
- US-A1- 2012 296 324
- US-A1- 2012 316 549
- US-A1- 2012 316 549
- US-A1- 2013 090 642
- US-A1- 2013 131 657
- US-B2- 7 116 870

## Description

### BACKGROUND OF THE INVENTION

### 1. TECHNICAL FIELD

The present invention relates to the field of surgery, and more particularly, to vessel manipulation.

### 2. DISCUSSION OF RELATED ART

Vessel manipulation is a commonly encountered challenge, especially in minimally invasive procedures. The variety of encountered vessels and the need to manipulate vessels without causing additional damage and bleeding require time and skill which may challenge procedure success and place a significant obstacle to the further development of such procedures.

U.S. Patent Application No. US 2012/296324 A1 teaches an energy-based instrument for sealing vascular tissue, comprising a deforming member configured to deform vascular tissue, and an optical system configured to illuminate a portion of the vascular tissue with light to seal the vascular tissue. The optical system comprising a light source configured to generate light, a light distribution element configured to distribute the light over the portion of the vascular tissue, and a light guide configured to guide the light from the light source to the light distribution element.

### SUMMARY OF THE INVENTION

The invention is defined in the appended claims. One aspect of the present invention provides surgical forceps comprising a vessel sealing tip, the vessel sealing tip comprising at least one energy delivering element arranged to deliver, upon actuation, energy to a vessel to yield a vessel welding effect in a specified sealing section of the vessel and to cut the vessel within the specified sealing section, wherein the at least one optical fiber comprises at least one specified emission region that is configured to emit transferred electromagnetic radiation from a core through a cladding of the optical fiber by the optical fiber being bent at the at least one specified emission region beyond a specified bending threshold.

These, additional, and/or other aspects and/or advantages of the present invention are set forth in the detailed description which follows; possibly inferable from the detailed description; and/or learnable by practice of the present invention.

### BRIEF DESCRIPTION OF THE DRAWINGS

For a better understanding of embodiments of the invention and to show how the same may be carried into effect, reference will now be made, purely by way of example, to the accompanying drawings in which like numerals designate corresponding elements or sections throughout.

In the accompanying drawings:
**Figures 1A-1C** are high level schematic illustrations of a vessel sealing tip for surgical forceps according to some embodiments of the invention.
**Figure 1D** is a high level schematic illustration of a fiber cross section according to some embodiments of the invention.
**Figures 2A****,** **2B** and **2C** are high level schematic illustrations of a vessel sealing tip for surgical forceps having focusing elements, according to some embodiments of the invention.
**Figures 3A** and **3B** are high level schematic illustrations of a vessel sealing tip for surgical forceps having vessel piercing elements, according to some embodiments of the invention.
**Figures 4A** and **4B** are high level schematic illustrations of a vessel sealing tip for surgical forceps having transversely expanding elements, according to some embodiments of the invention.
**Figure** 5 is a high level schematic illustration of a vessel sealing tip for surgical forceps enabling extension of the vessel sealing region, according to some embodiments of the invention.
**Figure** 6 is a high level schematic illustration of a vessel sealing tip for surgical forceps with variable intensity treatment, according to some embodiments of the invention.
**Figures 7A** and **7B** schematically illustrate surface designs that influence fiber bending, according to some embodiments of the invention.
**Figures 8A-8C** schematically illustrate fiber bending profiles, according to some embodiments of the invention.
**Figure** 9 is a high level schematic flowchart illustrating a vessel sealing method, according to some embodiments of the invention.

### DETAILED DESCRIPTION OF THE INVENTION

Prior to the detailed description being set forth, it may be helpful to set forth definitions of certain terms that will be used hereinafter.

The term "tissue" as used herein in this application refers to any bodily tissue, including vessels as defined below and any other type of tissue such as connective tissue, muscle tissue, nervous tissue, specific organs, fatty tissue, epithelial tissue and any combination thereof. The term "vessel" as used herein in this application refers to any bodily vessel, duct or tract. For example, the term "vessel" may refer to a blood vessel, a bile duct, an urinary tract or any other bodily vessel, duct or tract.

The terms "energy" or "treatment energy" as used herein in this application refer to any type of energy which is usable for treating or affecting vessels, for example electromagnetic energy in any form (e.g., optical energy, laser energy in any effective bandwidth, radiofrequency radiation - RF etc.), electrical or magnetic energy (e.g., electric currents or magnetic fields), ultrasonic radiation etc.

With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

Before at least one embodiment of the invention is explained in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

Embodiments of the invention provide a vessel sealing tip for surgical forceps which allows both sealing a vessel section and cutting therethrough without extracting the tip out of the body or exchanging the tip. Either a single action yields the sealing and the cutting, or two or more tip actions may be carried out sequentially to perform the sealing and cutting operations. In addition, the tip may be used for cutting through tissue. Embodiments of the tip may utilize any energy source, in particular optical laser energy but also RF or ultrasound energy. The different effects (sealing, cutting) may be achieved by varying the emitted energy spatially, by manipulating the vessel prior or during energy delivery, by changing a configuration of the tip during operation and by combining tensile forces or ablation at appropriate locations of the vessel. In certain embodiments, the vessel sealing tip may be used for general and robotic surgery. The disclosed devices may be used to achieve various tissue effects, e.g., coagulation, welding, sealing, cutting, ablation and combination thereof.

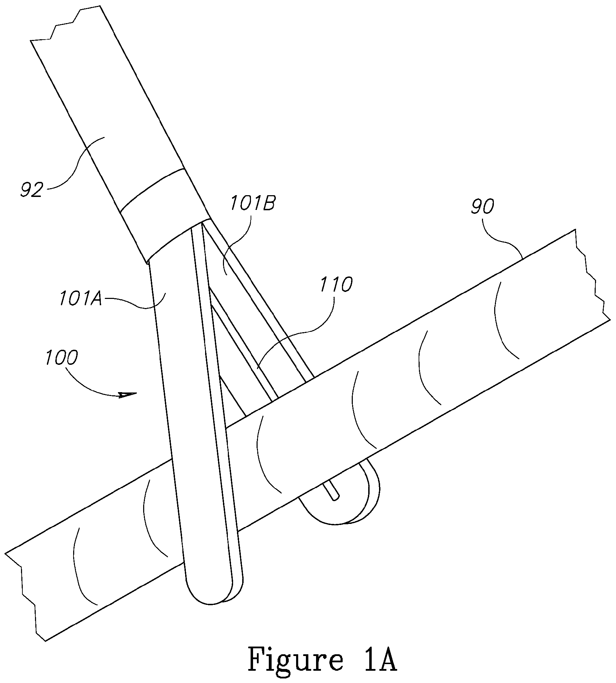

**Figures 1A-1C** are high level schematic illustrations of a vessel sealing tip **100** for surgical forceps **92** according to some embodiments of the invention.

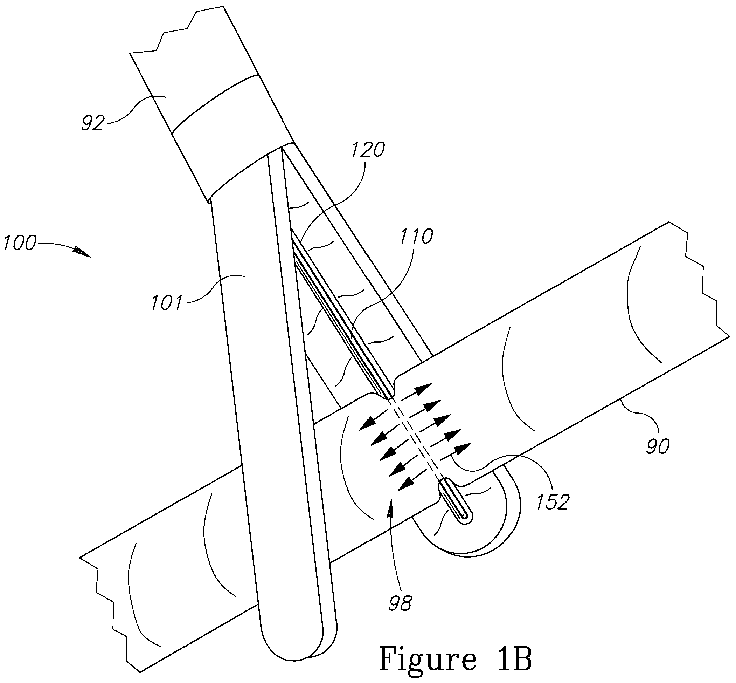

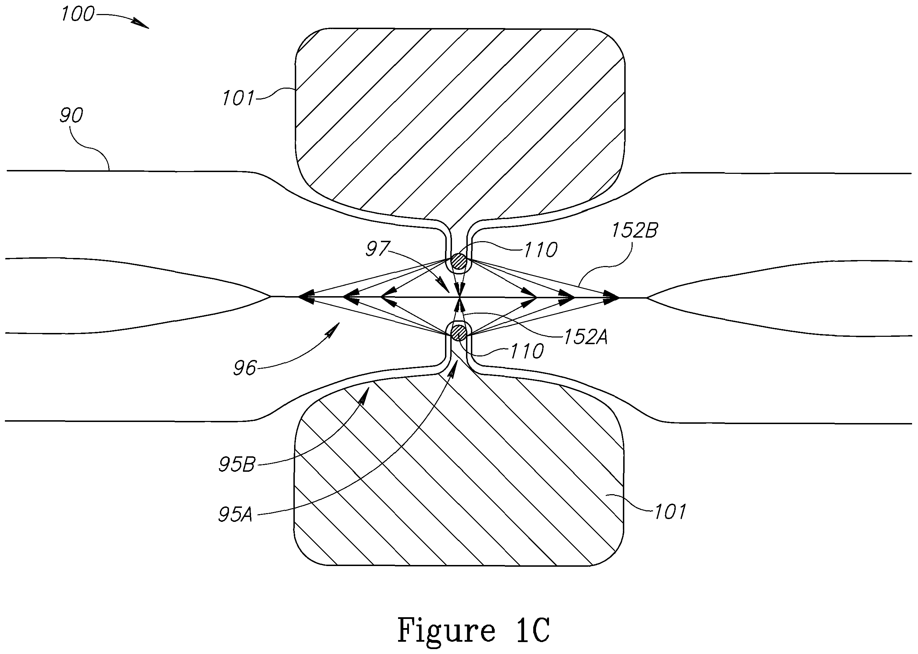

Vessel sealing tip **100** may comprise an energy delivery element **110** such as at least one optical element **110** arranged to deliver, upon actuation, electromagnetic radiation **152** to a vessel **90** to cut vessel **90** at a cutting region **98 (****Figure 1B****)** or to yield a vessel welding effect in a specified sealing section **96** of vessel **90 (****Figure 1C****),** and to cut vessel **90** at a cutting location **97** within specified sealing section **96.** For example, at least one optical element **110** may comprise at least one optical fiber **110** arranged to deliver electromagnetic radiation such as laser energy. In case of cutting region **98,** radiated energy **152** may also seal edges of the cut vessel during the cutting.

Energy delivery element **110** may be attached to any one of two jaws **101** (**101A, 101B**) of forceps tip **100,** or may also be a free element, at least on a part of the length thereof (see below).

In cases of energy delivery element **110** being an optical fiber, fiber **110** may emit radiation **152 (****Figure 1B****)** that yields a vessel sealing effect and radiation **152** that yields a vessel cutting effect. Radiation characteristics may be temporally varied in a controlled manner or may be designed in advance with respect to one or more vessel types. Radiation **152** may further be used to ablate the vessel wall prior to sealing and/or cutting vessel **110.** Radiation **152** may further be used to cut tissue during the advancement of tip **100;** for example, fiber **110** may continue beyond the illustrated extension to the very tip of either jaw **101,** to their external sides or may extend beyond tip **100** itself (e.g., form a loop ahead of tip **100).**

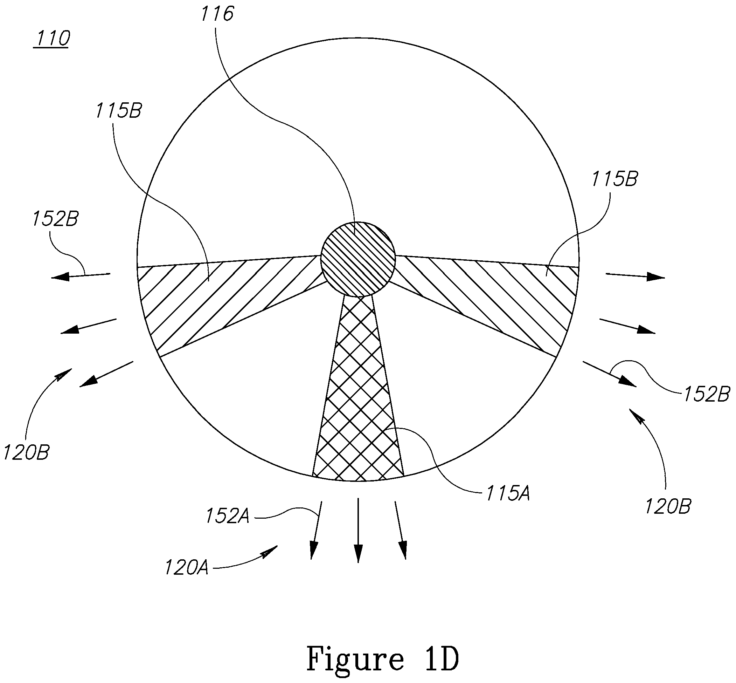

**Figure 1D** is a high level schematic illustration of a fiber cross section according to some embodiments of the invention. Fiber **110** may be arranged to emit at least two radiation types **152A, 152B** at at least two corresponding zones **(120A, 120B,** **Figures 1C****,** **1D)** of fiber **110.** The radiation types may differ in at least one of their intensity, spectral range, spatial and/or temporal patterns. Emission zones **120A, 120B** may comprise, in cross section, different corresponding fiber sectors **115A, 115B** made of different cladding materials or having different refractive indices. In embodiments, emission zones **120A, 120B** may have different cross-sections and thus different spatial energy density profiles. In certain embodiments, fiber **110** may comprise a solid core optical fiber (having core **116),** a hollow fiber or a photonic crystal fiber (such as a holey fiber, a Bragg fiber or any other micro-structured fiber). In certain embodiments, fiber **110** may comprise a metallic waveguide.

The different fiber sections may be differently micro-structured or have a different spatial arrangement of core **116** and cladding (e.g. core **116** may be asymmetrically positioned within the cladding). Fiber **110** may be single-mode or multi-mode. Beam polarization may also be used to differentiate radiation types **152A, 152B** and control the emitted energy density spatial distribution.

In certain embodiments, at least one jaw **101** of the forceps may comprise at least one protrusion **95A (****Figure 1C****)** arranged to constrict vessel **90** prior to the actuation of energy delivery element **110** (such as at least one optical element **110,** a RF source, an ultrasound source etc.). Protrusion **95A** protrudes from a surface **95B** of jaw **101** and constricts vessel **90** at the region of energy deliver to reduce the local thickness of vessel **90** and to provide more spatial variability in possible energy delivery directions. Energy delivery element **110** may be positioned fully or partially within protrusion **95A;** for example, at least one optical element **110** may be set within at least one protrusion **95A.**

Certain embodiments of the invention comprise a tip **100** with at least two jaws **101** for surgical forceps **92.** At least one of jaws **101** may comprise at least one protrusion **95A** positioned to contact tissue held by tip **100** and deliver both pressure and external energy to the tissue. The pressure may be a tip holding force (the force applied to the forceps and thereby transferred to the tip's jaws), concentrated by at least one protrusion **95A.** The external energy may be any of electromagnetic (e.g., optical, RF), electrical and ultrasound energy, or a combination thereof. At least one protrusion **95A** may comprise one or more thin element that concentrates applied forces onto a small section of vessel **90.** At least one protrusion **95A** may comprise an abrasive or an ablative element that reduces vessel wall thickness or even cuts the vessel, in addition to constricting the vessel.

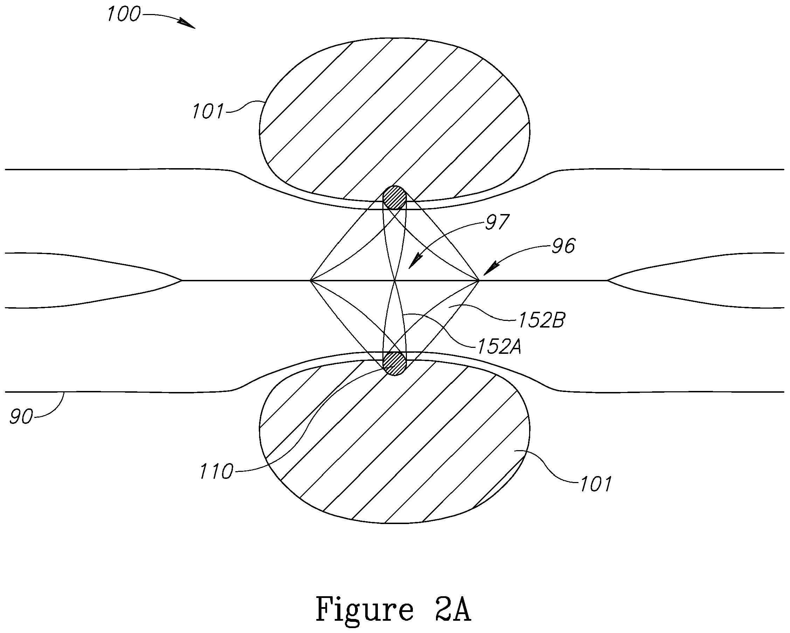

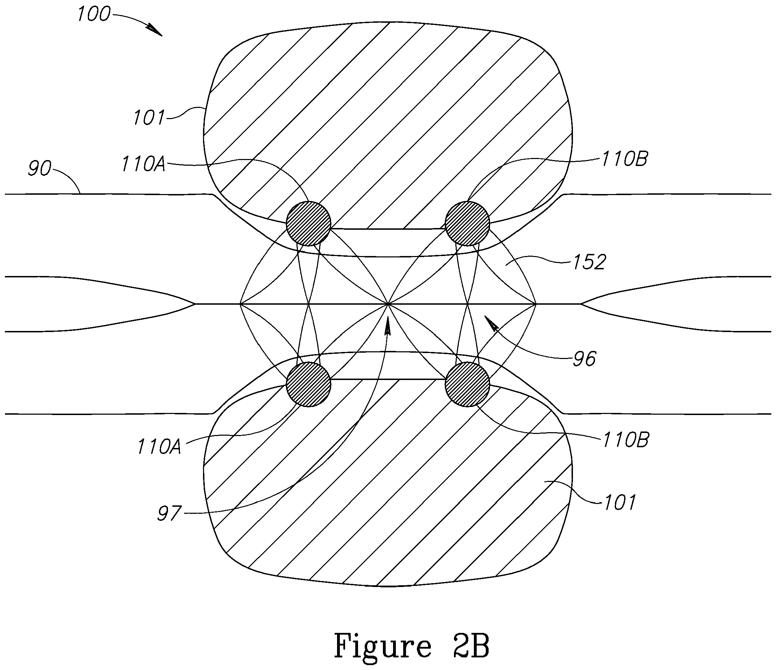

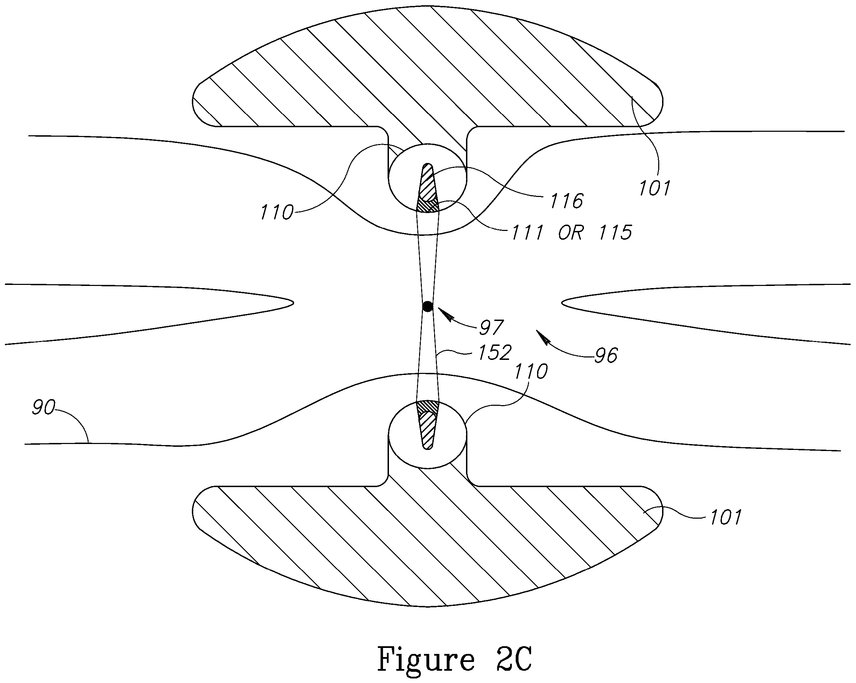

**Figures 2A****,** **2B** and **2C** are high level schematic illustrations of vessel sealing tip **100** for surgical forceps **92** having focusing elements **111,** according to some embodiments of the invention. Energy delivery elements **110** may comprise focusing elements **111 (****Figure 2C****)** arranged to focus any type of delivered energy (e.g., optical energy, RF, ultrasound, electrical energy, etc.). For example, optical elements **110** may comprise at least one focusing element **111,** such as a lens **111** or a sector **115** of the cladding arranged to focus emitted radiation **152.** In the non-limiting example illustrated in **Figure 2C****,** a combination of asymmetric core **116** and focusing element **111** may be arranged to yield the welding and/or cutting of vessel **90** (depending on the delivered radiation and tip manipulation). The focusing of the emitted radiation may be in a cross-sectional plane of optical element **110.** In certain embodiments, focusing elements **111** may focus different types of radiation **152A, 152B** at different regions of vessel **90,** e.g., radiation **152B** may be focused to produce a sealing effect at sealing region **96,** and radiation **152A** may be focused to cut vessel **90** at cutting region **97.** Focusing elements **111** may be embedded in or attached to forceps jaws **101.** Focusing elements **111** may be multiply associated with at least one of jaws **101,** as illustrated in **Figure 2B****.** The focusing elements may be embedded in multiple fibers **110A, 110B** and be arranged to jointly apply the sealing and cutting to regions **96, 97** of vessel **90** respectively. Particularly, at least two focusing elements **111** may be positioned on each jaw **101** and arranged to yield a specified extension of specified sealing section **96,** which is broader than sealing section **96** produced by a single focusing element **111** or solely by optical element **110.**

In certain embodiments, energy delivery element **110** may be arranged to reduce a vessel wall thickness prior to the welding. For example, optical element **110** may operate in an ablative mode to reduce vessel wall thickness prior of holding vessel **90** sealing it and cutting through vessel **90.** The reduction of wall thickness allows energy to be delivered to the internal walls of vessel **90** without causing thermal damage to the external wall of vessel **90.** Furthermore, reducing the wall thickness may reduce the wall resistance to mechanical pressure and thus allow a more effective application of pressure to vessel **90,** e.g., by protrusions **95A (****Figure 1C****),** to create a more effective gripping of vessel **90** by forceps **92** and a better sealing effect.

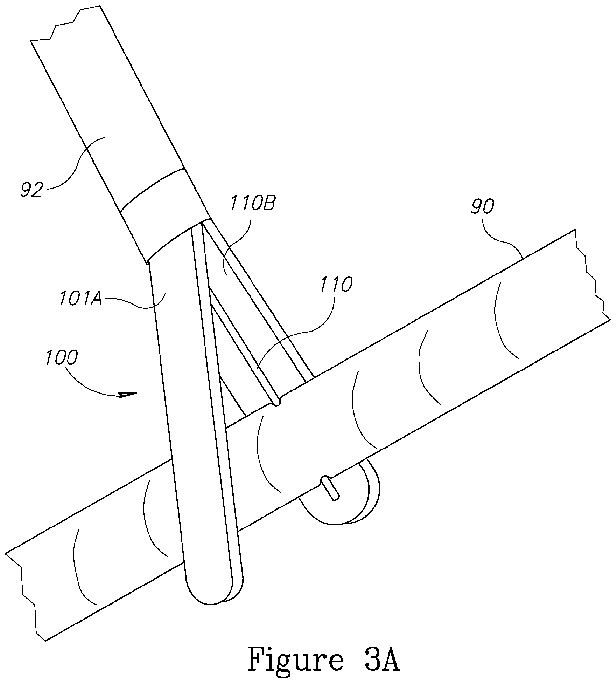

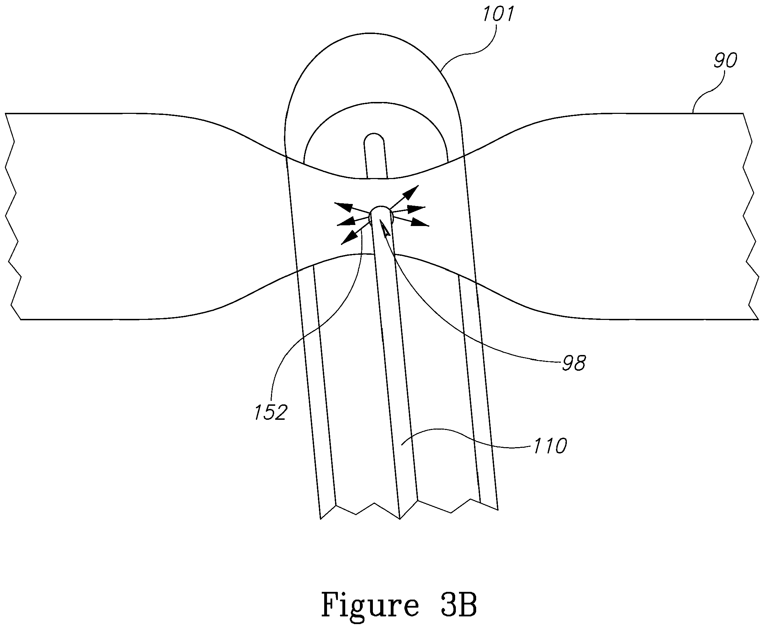

**Figures 3A** and **3B** are high level schematic illustrations of vessel sealing tip **100** for surgical forceps **92** having vessel piercing elements **110,** according to some embodiments of the invention. Energy delivery element **110** may comprise at least one optical element **110** comprising at least one optical fiber **110** arranged to penetrate a lumen of vessel **90,** piercing thereby a hole **98** in vessel **90,** prior to the actuation thereof and emission of energy **152** from fiber **110.** In certain embodiments, penetrating the vessel lumen enables more efficient sealing and/or cutting of vessel **90.** Delivering energy from the interior of vessel **90** allows treating its inner layers directly, without having to apply high pressure on the vessel wall in order to flatten vessel **90.** In certain embodiments, penetrating and flattening may be applied simultaneously or sequentially to reciprocally enhance the sealing effect.

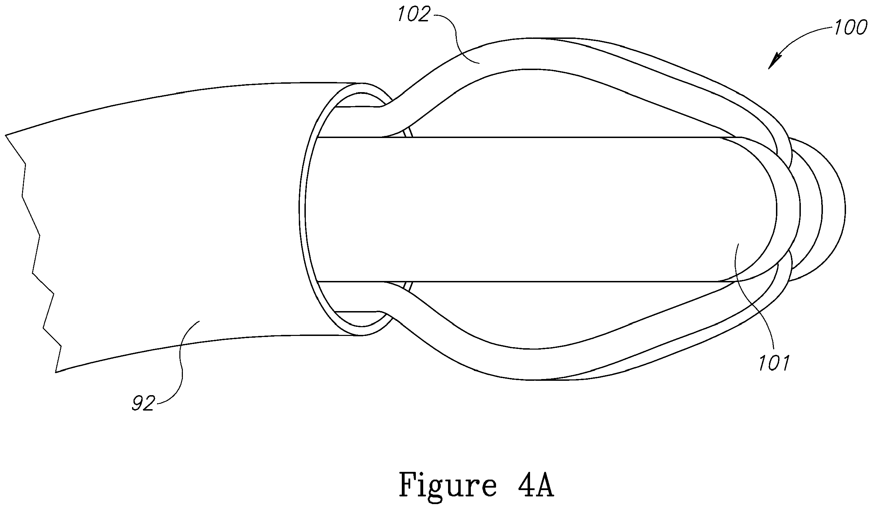

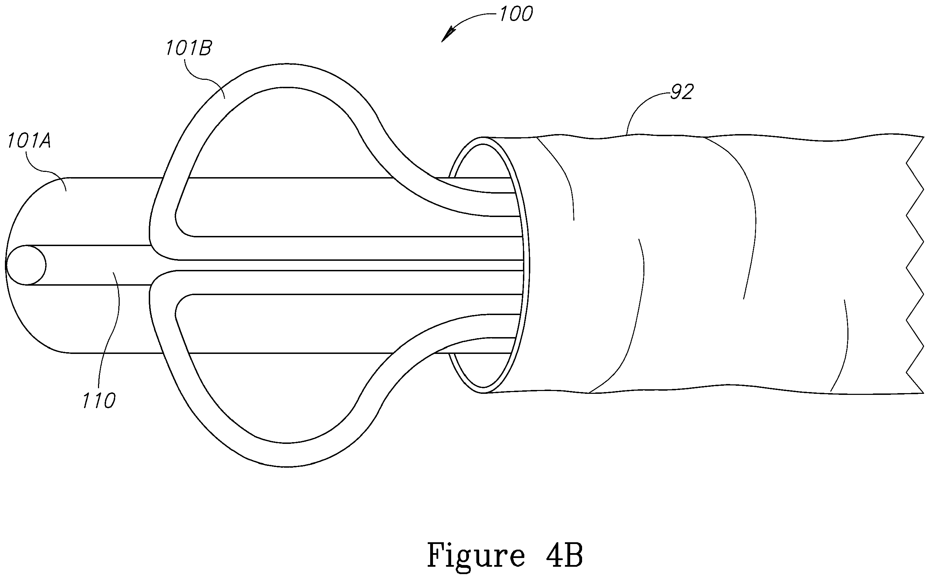

**Figures 4A** and **4B** are high level schematic illustrations of vessel sealing tip **100** for surgical forceps **92** having transversely expanding elements **102,** according to some embodiments of the invention. At least one jaw **101** of tip **100** may comprise transversely expandable element **102** arranged to yield a specified extension of specified sealing section **96.** Mechanically pressing a wider section of vessel **90** increases the potential sealing section of vessel **90** and hence may improve sealing and vessel manipulation conditions. Transversely expanding elements **102** may be retracted in a tool delivery channel and expand only in situ, upon using tip **100.** In certain embodiments, transversely expanding elements **102** are controllably expandable, e.g., by a user of forceps **92** or responsive to applied pressure on respective jaw **101** or sensed resistance of vessel **90.** In certain embodiments, transversely expanding elements **102** may mechanically extend specified sealing section **96** during the welding. The welding (e.g., by radiation **152B** aimed at sealing section **96)** may be combined with transverse forces applied to vessel **90** at the sealing section and aimed to expand the treated section. A reduced resistance of vessel **90** due to the energy radiation and/or the mechanical extending may thus be exploited to expand the sealing section. In certain embodiments **(****Figure 4B****),** at least one of jaws **101** may be designed as a transversely expanding element. For example, one jaw **101A** may be a regular jaw and the other jaw **101B** may be expandable to broaden the sealing region of vessel **90.** Expandable jaw **101B** may be made of two or more parts and tip **100** may comprise means to separate the parts to further enhance the stretching effect on vessel **90.**

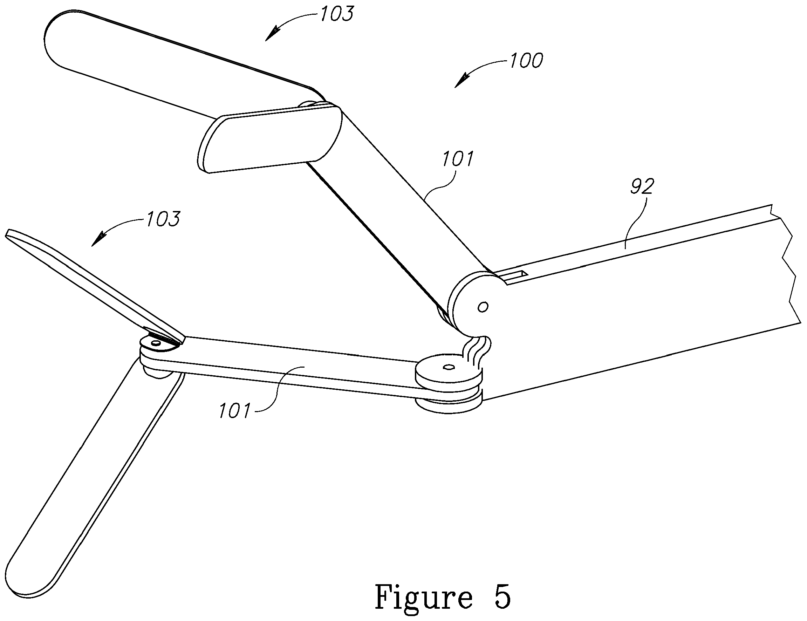

**Figure 5** is a high level schematic illustration of vessel sealing tip **100** for surgical forceps **92** enabling extension of the vessel sealing region, according to some embodiments of the invention. In certain embodiments, one or both jaws **101** may be hingedly attached to forceps **92** and may be controllably pivotally movable to stretch vessel **90,** e.g., during sealing or cutting thereof. One or both jaws **101** may comprise a forceps element **103** arranged to hold and/or pull a respective vessel section to generate the stretching effect of the vessel, which expands the sealing region. Jaws **101** may be moveable along different spatial directions, to yield an additional twist of vessel **90,** selected to further enhance the stretching of the sealing region. Any of the above mentioned movements and actions may be combined with energy delivery to enhance the sealing and/or cutting effect. Accordingly, control of any of these movements may be carried out by a user or responsive to sensed forces in tip **100.** Jaws **101** may by controlled by mechanical compliance to exerted forces.

In certain embodiments, vessel sealing tip **100** for surgical forceps **92** may comprise at least one transversely expandable element **110** or **103** arranged to yield a specified extension of a specified section of vessel **90** and energy delivery element **110** arranged to deliver external energy, upon actuation, to vessel **90** to yield a vessel welding effect in a specified sealing section of vessel **90** and to cut vessel **90** within the specified sealing section. The external energy may be at least one of optical, electrical and ultrasound energy. Tip **100** may thus open up and create a seal larger than half width of tool (e.g., tip **100)** or just separate regions of cut and seal. The specified sealing section may be mechanically extended during the welding with or without additional energy delivery. In embodiments, tip **100** may comprise two transversely expandable elements **103,** each arranged to yield a specified extension of the specified section of vessel **90** in a different plane.

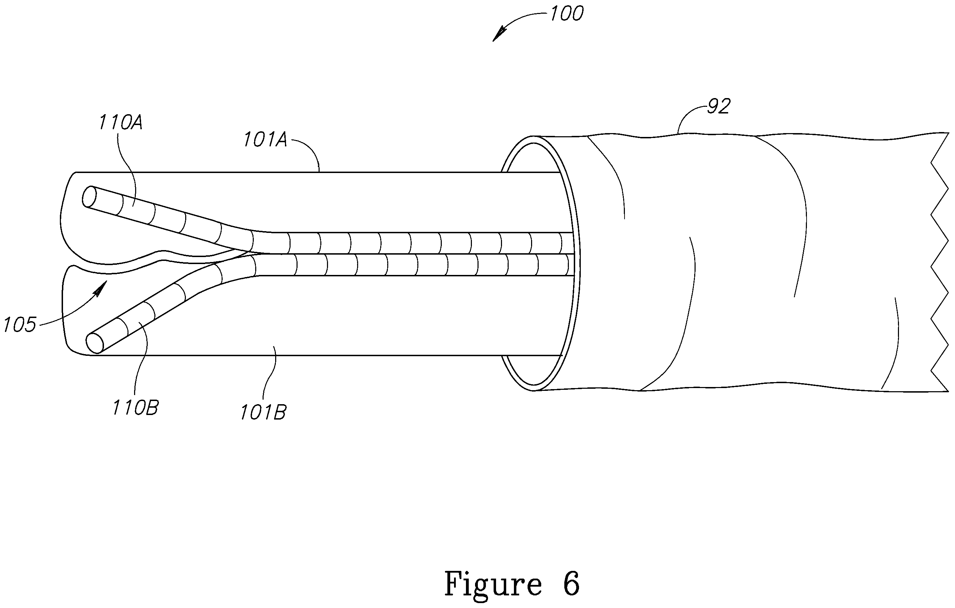

**Figure 6** is a high level schematic illustration of vessel sealing tip **100** for surgical forceps **92** with variable intensity treatment, according to some embodiments of the invention. In certain embodiments, each jaw **101A, 101B** of the forceps may comprise at least one optical fiber **110A,** 110B respectively, positioned at a distance from an edge **105** of the respective jaw, wherein the distance may vary along the jaws. For example, the varying distance with respect to treating edge **105** of the jaws may diminish from a tip to a base of jaws **101A, 101B** to yield the welding effect at the jaw tip and perform the cutting between the tip and the base of the jaw. In certain embodiments, the sealing and cutting effects are thus spatially differentiated along the jaws instead or in addition to the spatial differentiation across the jaws illustrated previously (cf., e.g., **Figure 2A****).** In certain embodiments, one or more fibers **110A, 110B** may have longitudinally varying characteristics that generate radiation **152** of different characteristics along the fiber. For example, radiation **152** designed for sealing may be applied at the jaw tips where the distance to vessel **90** is also the largest, and radiation **152** designed for cutting may be applied at the jaw bases where the distance to vessel **90** is also the smallest. Hence jaws **101** and energy delivery elements **110** may be designed to jointly differentiate welding and cutting effects.

In certain embodiments, vessel sealing tip **100** may be constructed from non-metallic materials to allow use of tip **100** simultaneously with MRI imaging. For example, tip **100** may be made of plastic and energy may be delivered via optical fibers.

In certain embodiments, vessel sealing tip **100** may comprise at least one wave guide (not shown) arranged to deliver, upon actuation, electromagnetic radiation to the vessel to yield a vessel welding effect in a specified sealing section of the vessel and to cut the vessel within the specified sealing section. In certain embodiments, at least one jaw of the forceps may comprise at least one protrusion arranged to constrict the vessel prior to the actuation of the at least one wave guide.

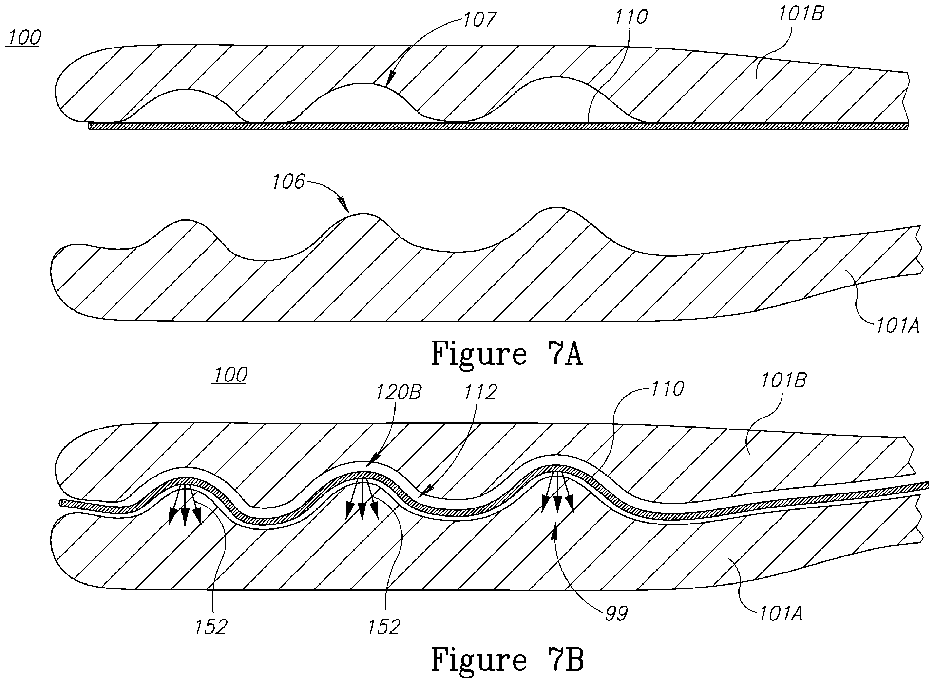

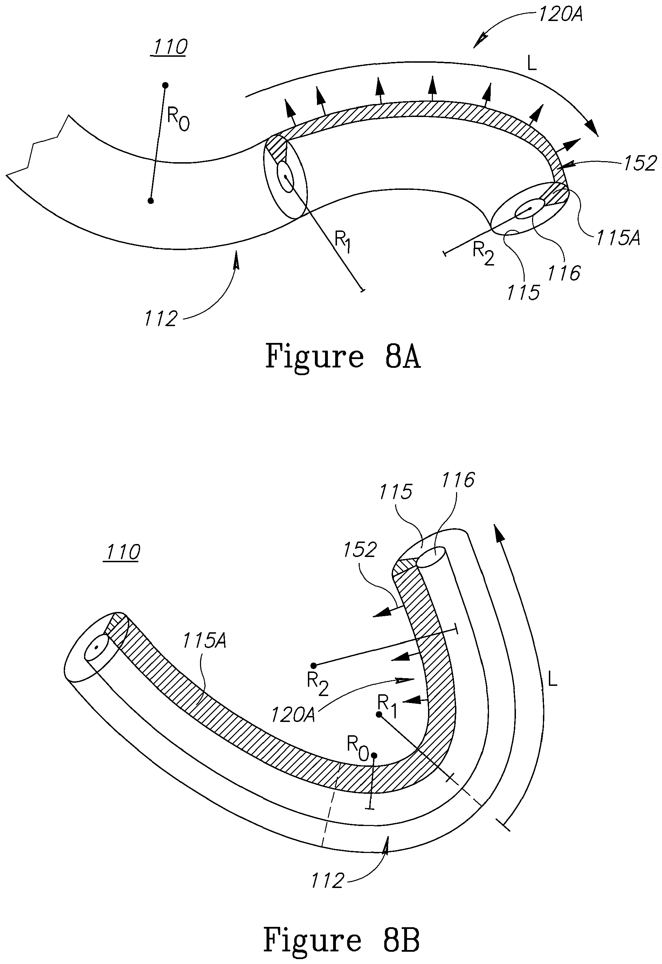

**Figures 7A** and **7B** schematically illustrate surface designs that influence fiber bending, according to some embodiments of the invention. **Figures 7A** and **7B** illustrate, respectively, an open, non-emitting, position and an active position of tweezers-like device **100,** e.g., vessel sealing tip **100.** Fiber **110** is integrated within tweezers device **100** in a way that causes bending of fiber **110** upon handling tissue with device **100** and radiation emission from the bended regions **120B** which enhances treatment of the handled tissue. For example, fiber **110** may be associated with one arm **101B** of tweezers device **100** and fiber bending may occur upon pressing the fiber against a second arm **101A** of tweezers device **100.** Any of the tweezers' arms may comprise protrusions **106** and/or corresponding recesses **107** to enhance fiber bending upon handling tissue by tweezers **100** and thereby yield treatment at regions **99** which may be sealing regions **96** and/or cutting regions **97, 98.** Protrusions **106** and/or corresponding recesses **107** may be further designed to define pre-bend regions **112** as explained below **(****Figures 8A-8C****).** Tweezers-like device **100** may comprises surface features designed to control the bending of optical fiber **110** upon tissue contact. For example, tweezers device **100** may have multiple fibers **110** which may have differing emission characteristics, e.g., configured to apply different effects to the treated tissue, and/or tweezers device **100** may have multiple types of protrusions **106** and recesses **107** having different curvatures and hence determining different types of emitted radiation and respective effects on the tissue. Tweezers device **100** hence allows mechanical handling while using laser for welding and/or cutting tissue. The emission may be dependent on the extent of the force applied by the physician through the extent of resulting bending of fiber(s) **110.** The closer arms **101A, 101B** are pressed together, the larger becomes the fiber bending and the emitted radiation.

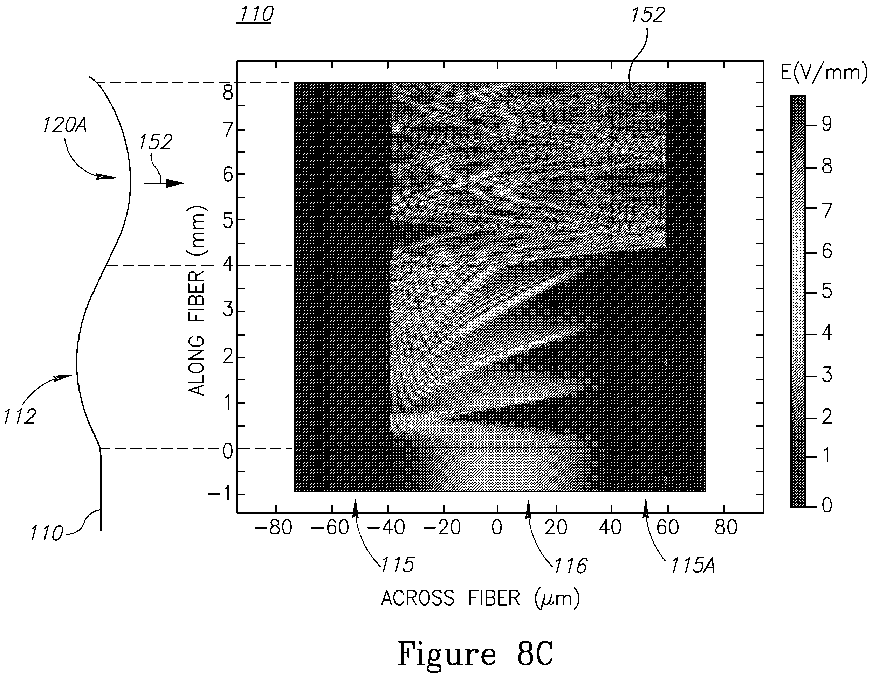

**Figures 8A-8C** schematically illustrate fiber bending profiles, according to some embodiments of the invention. **Figures 8A** and **8B** schematically illustrate two types of fiber bending and corresponding emission zones **120A,** while **Figure 8C** illustrates simulation results that exemplify the configuration of pre-bend region **112.** Both **Figures 8A** and **8B** illustrate pre-bend region **112** and **Figure 8C** illustrates the operation of pre-bend region **112.**

**Figure 8A** schematically illustrates fiber **110** having emitting zone **115A** at emission region **120A,** emitting radiation **152** outwardly from convex emission region **120A.** Emission region **120A** is characterized by a decreasing radius of curvature R₂<R₁ along its length L (the arrow marks the direction of radiation propagation) and generally dR/dL<0 along emission region **120A.** Emission region **120A** is preceded by pre-bend region **112** having an opposite curvature (R₀) and configured to enhance emission from emitting zone **115A** upon the bending of emission region **120A.** Protrusions **106** and/or recesses **105** in supportive structures may be used to define the changing of the radius of curvature mechanically, or the radius may be changed by the practicing physician manually or electronically.

**Figure 8B** schematically illustrates fiber **110** having emitting zone **115A** at emission region **120A,** emitting radiation **152** inwardly from concave emission region **120A.** Emission region **120A** is characterized by an increasing radius of curvature R₂>R₁ along its length L (the arrow marks the direction of radiation propagation) and generally dR/dL>0 along emission region **120A.** Emission region **120A** is preceded by pre-bend region **112** having still stronger curvature R₀<R₁ and configured to enhance emission from emitting zone **115A** upon the bending of emission region **120A.** Protrusions **106** and/or recesses **105** in supportive structures may be used to define the changing of the radius of curvature mechanically, or the radius may be changed by the practicing physician manually or electronically. Other techniques may be used to yield and use inwards emission by bending the fiber.

**Figure 8C** schematically illustrates simulation results that exemplify the functioning of pre-bend region **112** in the configuration illustrated in **Figure 8A****.** The electromagnetic finite elements simulation illustrates a cross section of fiber **110** along pre-bend region **112** and emission region **120A.** The x axis is across fiber **110** (-75µm to +75µm), including non-emitting cladding zone **115** (-75µm to -40µm), core **116** (-40µm to +40µm) and emitting cladding zone **115A** (+40µm to +75µm). It is noted that the simulated fiber is actually bent as illustrate to the left of the simulation results, while simulation results are shown for convenience along a seemingly straight fiber, using a conformal mapping technique. The y axis is along fiber **110** (-1mm to +8mm), including a straight (not bended) fiber region (-1mm to 0mm), pre-bend region **112** (0mm to 4mm) and emitting region (4mm to 8mm). Radiation intensity (simulated norm strength of the electric field) is indicated by gray levels from zero (black) to ca. 6000 (white) Volts/m and beyond (larger values are scarcely represented in the illustration and are seen as black streaks at the very edge of the intensity maxima at ca. +0.5mm and 2mm, left to the center of the core). While intensity distribution along central non-bent region is Gaussian about the center of core **116,** pre-bend region **112** exhibits higher propagation modes and energy concentration at the left, convex side of the bending. No emission is observed from the fiber due to the high energy barrier in the direction opposing the emission zone. The pre-bend radius R₀ may be large or small, without resulting in emission. Upon bending the fiber to the other direction at emitting region **120A,** the eccentrically distributed energy is emitted through respective cladding zone **115A.** Bending radii R₀, R₁, R₂ and the refractive indices of core **116,** non-emitting cladding **115** and emitting cladding zone **115A** are configured according to specified required rending configurations and performance requirements. For the configuration illustrated in **Figure 8B****,** similar eccentric concentration of energy occurs along pre-bend region **112,** but energy is released through emission zone **115A** upon increasing the radius of curvature and utilizing the change in energy distribution that is illustrated in **Figure 8C** in the regions between 4-4.5mm and shows a tendency of the energy distribution to move to the right side of the fiber upon increasing the curvature radius. Clearly, the refractive indices are configured to enhance this effect.

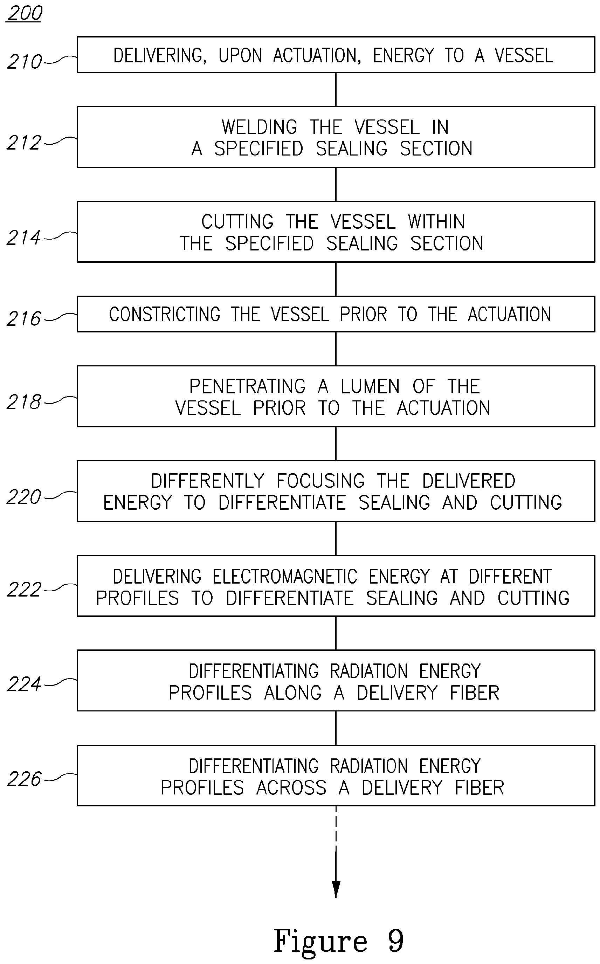

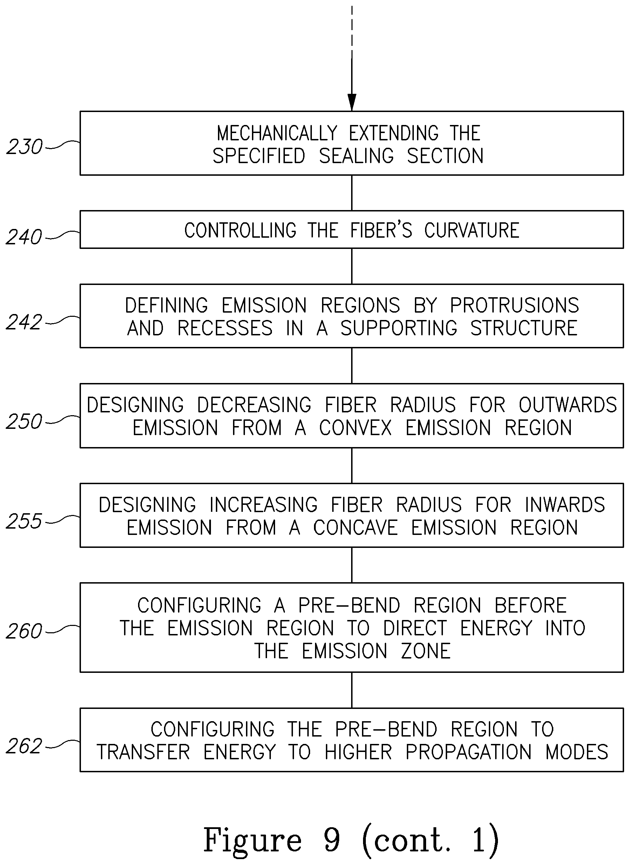

**Figure 9** is a high level schematic flowchart illustrating a vessel sealing method **200,** according to some embodiments of the invention. Method **200** may be used to achieve different tissue effects, ranging from welding through sealing to cutting, ablation and any combination of these and other effects (e.g., coagulation).

Vessel sealing method **200** comprises delivering, upon actuation, energy to a vessel (stage **210)** to yield a vessel welding effect in a specified sealing section of the vessel and to cut the vessel within the specified sealing section. Method **200** may comprise welding the vessel in a specified sealing section (stage **212)** and cutting the vessel within the specified sealing section (stage **214).** In certain embodiments, the welding and the cutting may be carried out by a single actuation. The delivered energy may comprise at least one of optical, electrical and ultrasound energy

For example, the delivered energy may be electromagnetic radiation and method **200** may further comprise creating the welding and cutting by differently focusing the delivered electromagnetic radiation on the specified sealing section and on the cutting location, respectively, to differentiate sealing and cutting (stage **220).**

In another example, the delivered energy may be electromagnetic radiation method **200** may further comprise using at least one optical fiber arranged to emit the electromagnetic radiation at at least two radiation profiles, one corresponding to welding **212** and another corresponding to cutting **214** the vessel. Generally, certain embodiments may comprise delivering electromagnetic energy at different profiles to differentiate sealing and cutting (stage **222).** For example, radiation energy profiles may be differentiated along a delivery fiber (stage **224),** across a delivery fiber (stage **226)** or by a combination thereof and in respect to the positioning of the delivery fibers in jaws of a forceps tip arranged to perform method **200.**

In certain embodiments, method **200** may further comprise constricting the vessel prior to the actuation (stage **216).** The constriction may be arranged to yield more effective sealing and/or cutting by reducing the vessel diameter and increasing the usable spatial variability of energy delivery.

In certain embodiments, method **200** may further comprise penetrating a lumen of the vessel prior to the actuation (stage **218).** Penetrating the vessel enables sealing the vessel from within and thereby applying the delivered energy efficiently and in a controllable manner to seal and cut the vessel.

In certain embodiments, method **200** may further comprise mechanically extending the specified sealing section (stage **230).** The extending may be carried out prior, during or after sealing the vessel to broaden the sealing section to allow more effective cutting and healing of the cutting location.

Method **200** may further comprise controlling the fiber's curvature (stage **240),** for example by defining emission regions by protrusions and recesses in a supporting structure (stage **242)** such as forceps, tweezers or any other structure.

Method **200** may comprise configuring emission from an optical fiber by arranging at least one specified region in the optical fiber to emit transferred electromagnetic radiation from a core through a cladding of the optical fiber upon bending of the optical fiber at the at least one specified region beyond a specified bending threshold.

Method **200** may comprise designing decreasing fiber radius for outwards emission from a convex emission region (stage **250).** Alternatively or additionally, method **200** may comprise designing increasing fiber radius for inwards emission from a concave emission region (stage **255).** Protrusions and/or recesses in the supportive structure may be used to define the changing of the radius of curvature mechanically.

In certain embodiments, method **200** may further comprise configuring a pre-bend region before the emission region to direct energy into the emission zone (stage **260)** and/or configuring the pre-bend region to transfer energy to higher propagation modes (stage **262).**

In certain embodiments, vessel sealing tip **100** for surgical forceps **92** may be configured to be applied for any of the following treatments: Sealing blood vessels, arteries, veins; Sealing biliary ducts; Sealing urinary tract; Sealing reproductive tract; Sealing airways; Sealing in the GI tract; Sealing the dura; Treating septums (nasal, atrial, etc.); Sealing organs such as lung, liver, spleen, heart, stomach, pancreas, uterus, bladder, kidney etc. While the above description mainly referred to treating vessels **90,** tip **100** for surgical forceps **92** may be configured for treating any other type of tissue, as well as to carry out further surgical tasks, such as cutting or ablating tissue.

In a non-limiting example, vessel sealing tip **100** may be configured to apply pressures in the at least a part of the range 20-400 PSI. The outer diameter of fiber(s) **110** may be between 0.05-2mm and fiber(s) **110** may be arranged to deliver power levels between e.g. 1W-100W. Tip **100** may be configured to have a jaw **101** length between 2-50mm, a jaw **101** width between 0.5-10mm, and a ridge width of at least one protrusion **95A** between 0.1-5mm. The dimensions of jaws **101** may be configured with respect to the specific use of tip **100,** as illustrated in the examples above. For example, larger tips **100** may be designed to seal larger or stiffer vessels **90.**

**Table 1** is a non-limiting exemplary overview of possible tip characteristics for various applications of tip **100.**

**Table 1: Tip parameters for various applications**

| Vessel type (Sealing operation, unless otherwise indicated) | Anatomical size (mm) | Jaw length (mm) | Jaw width (mm) | Working area length (mm) |
|---|---|---|---|---|
| Blood vessels, arteries, veins | <1 to 10 | 20 and up | 2 to 10 | 17 and up |
| Extremely large blood vessel, aorta, aneurisms, etc. | up to 25 | 40 to 60 | 2 to 10 | 35 and up |
| Biliary ducts | 5 -10 | 20 and up | 2 to 10 | 8 and up |
| Urinary tract | up to 10 | 20 and up | 2 to 10 | 17 and up |
| Reproductive tract | Fallopian tubes up to 2, general tissue much more | 20 and up | 2 to 10 | 17 and up |
| Airways | | 20 and up | 2 to 10 | 17 and up |
| GI tract | | 30 and up | 2 to 10 | 25 and up |
| Dura | | 20 and up | 2 to 10 | 15 and up |
| Septum (nasal, atrial, etc.) | | 3 and up | 1 to 10 | 2 and up |
| Operating on organs such as lung, liver, spleen, heart, stomach, pancreas, uterus, bladder, kidney, etc. | | 20 and up | 2 to 10 | 17-70 |
| Neurological operations | | 3 and up | 2 to 10 | 3 and up |
| Kidney operations | | 20 and up | 2 to 10 | 17 and up |

In the above description, an embodiment is an example or implementation of the invention. The various appearances of "one embodiment", "an embodiment", "certain embodiments" or "some embodiments" do not necessarily all refer to the same embodiments.

Although various features of the invention may be described in the context of a single embodiment, the features may also be provided separately or in any suitable combination. Conversely, although the invention may be described herein in the context of separate embodiments for clarity, the invention may also be implemented in a single embodiment.

Certain embodiments of the invention may include features from different embodiments disclosed above, and certain embodiments may incorporate elements from other embodiments disclosed above. The disclosure of elements of the invention in the context of a specific embodiment is not to be taken as limiting their used in the specific embodiment alone.

Furthermore, it is to be understood that the invention can be carried out or practiced in various ways and that the invention can be implemented in embodiments other than the ones outlined in the description above.

The invention is not limited to those diagrams or to the corresponding descriptions. For example, flow need not move through each illustrated box or state, or in exactly the same order as illustrated and described.

Meanings of technical and scientific terms used herein are to be commonly understood as by one of ordinary skill in the art to which the invention belongs, unless otherwise defined.

While the invention has been described with respect to a limited number of embodiments, these should not be construed as limitations on the scope of the invention, but rather as exemplifications of some of the preferred embodiments. Other possible variations, modifications, and applications are also within the scope of the invention. Accordingly, the scope of the invention should not be limited by what has thus far been described, but by the appended claims and their legal equivalents.

## Claims

1. Surgical forceps (92) comprising a vessel sealing tip (100), the vessel sealing tip (100) comprising at least one optical fiber (110) arranged to deliver, upon actuation, electromagnetic radiation (152) to a vessel (90) to yield a vessel welding effect in a specified sealing section (96) of the vessel (90) and/or to cut the vessel (90) within the specified sealing section (96), wherein the at least one optical fiber (110) comprises at least one specified emission region (120A) that is configured to emit transferred electromagnetic radiation from a core (116) through a cladding (115A) of the optical fiber (110) by the optical fiber (110) being bent at the at least one specified emission region (120A) beyond a specified bending threshold.

2. The surgical forceps of claim 1, wherein at least one jaw (101) of the forceps (92) comprises at least one protrusion (95A) arranged to constrict the vessel (90) prior to the actuation of the at least one optical fiber (110).

3. The surgical forceps of claim 1, comprising focusing elements arranged to focus delivered energy.

4. The surgical forceps of claim 1, wherein the vessel sealing tip (100) further comprises at least one focusing element (111) arranged to focus emitted radiation (152) in a cross-sectional plane of the at least one optical fiber (110).

5. The surgical forceps of claim 1, wherein at least one optical element comprising the at least one optical fiber (110) is configured to operate in an ablative mode to reduce a vessel wall thickness prior to the welding and/or cutting, or
wherein the forceps comprise jaws (101), wherein at least one of the jaws (101) comprises at least one protrusion (95A) positioned to contact tissue held by the vessel sealing tip (100), said protrusion comprising an abrasive or ablative element configured to reduce a vessel wall thickness or to cut the vessel.

6. The surgical forceps of claim 1, wherein at least one jaw (101) of the forceps (92) comprises a transversely expandable element (102) arranged to yield a specified extension of the specified sealing section (96).

7. The surgical forceps of claim 1, further arranged to mechanically extend the specified sealing section (96) during the welding.

8. The surgical forceps of claim 1, wherein the vessel sealing tip is constructed from non-metallic materials.

9. The surgical forceps of claim 2, wherein the at least one optical fiber (110) is set within the at least one protrusion (95A).

10. The surgical forceps of claim 4, wherein the focusing of the emitted radiation is in a cross-sectional plane of the at least one optical fiber (110).

11. The surgical forceps of claim 4, further comprising at least two focusing elements (111) positioned and arranged on each jaw (101) to yield a specified extension of the specified sealing section (96).

## Patentansprüche

1. Chirurgische Zange (92), die eine Gefäßversiegelungsspitze (100) umfasst, die Gefäßversiegelungsspitze (100) umfasst mindestens eine Glasfaser (110), die angeordnet ist, um bei Betätigung elektromagnetische Strahlen (152) an ein Gefäß (90) zu senden, um einen gefäßverschweißenden Effekt in einem spezifischen Versiegelungsabschnitt (96) des Gefäßes (90) zu erreichen und/oder um das Gefäß (90) innerhalb des spezifischen Versiegelungsabschnitts (96) zu schneiden, wobei die mindestens eine Glasfaser (110) mindestens einen spezifischen Emissionsbereich (120A) umfasst, der konfiguriert ist, um übertragene elektromagnetische Strahlung von einem Kern (116) durch eine Umhüllung (115A) der Glasfaser (110) abzugeben, wobei die Glasfaser (110) in dem mindestens einen spezifischen Emissionsbereich (120A) über eine spezifische Biegeschwelle gebogen wird.

2. Die chirurgische Zange nach Anspruch 1, wobei mindestens eine Backe (101) der Zange (92) mindestens einen Vorsprung (95A) umfasst, der angeordnet ist, um das Gefäß (90) vor der Betätigung der mindestens einen Glasfaser (110) zusammenzuziehen.

3. Die chirurgische Zange nach Anspruch 1, die Fokussierungselemente umfasst, um die gelieferte Energie zu fokussieren.

4. Die chirurgische Zange nach Anspruch 1, wobei die Gefäßversiegelungsspitze (100) ferner mindestens ein Fokussierungselement (111) umfasst, das angeordnet ist, um die abgegebene Strahlung (152) auf einer Querschnittsebene der mindestens einen Glasfaser (110) zu fokussieren.

5. Die chirurgische Zange nach Anspruch 1, wobei mindestens ein optisches Element, das die mindestens eine Glasfaser (110) umfasst, konfiguriert ist, um in einem ablativen Modus betrieben zu werden und eine Gefäßwanddicke vor dem Verschweißen und/oder Schneiden zu reduzieren, oder
wobei die Zange Backen (101) umfasst, wobei mindestens eine der Backen (101) mindestens einen Vorsprung (95A) umfasst, der positioniert ist, um das von der Gefäßversiegelungsspitze (100) gehaltene Gefäß zu halten, dieser Vorsprung umfasst ein abrasives oder ablatives Element, das konfiguriert ist, um eine Gefäßwanddicke zu reduzieren oder das Gefäß zu schneiden.

6. Die chirurgische Zange nach Anspruch 1, wobei mindestens eine Backe (101) der Zange (92) ein quer ausfahrbares Element (102) umfasst, das angeordnet ist, um eine spezifische Erweiterung des spezifischen Versiegelungsabschnitts (96) zu erreichen.

7. Die chirurgische Zange nach Anspruch 1, die ferner angeordnet ist, um den spezifischen Versiegelungsabschnitt (96) während des Verschweißens mechanisch zu erweitern.

8. Die chirurgische Zange nach Anspruch 1, wobei die Gefäßversiegelungsspitze aus nicht-metallischen Materialien hergestellt ist.

9. Die chirurgische Zange nach Anspruch 2, wobei die mindestens eine Glasfaser (110) innerhalb des mindestens einen Vorsprungs (95 A) untergebracht ist.

10. Die chirurgische Zange nach Anspruch 4, wobei die Fokussierung der abgegebenen Strahlung auf einer Querschnittsebene der mindestens einen Glasfaser (110) stattfindet.

11. Die chirurgische Zange nach Anspruch 4, die ferner mindestens zwei Fokussierungselemente (111) umfasst, die an *jeder Backe* (101) positioniert und angeordnet sind, um eine spezifische Erweiterung des spezifischen Versiegelungsabschnitts (96) zu erreichen.

## Revendications

1. Forceps chirurgical (92) comprenant un embout d'obturation de vaisseau (100), l'embout d'obturation de vaisseau (100) comprenant au moins une fibre optique (110) agencée pour délivrer, après son actionnement, un rayonnement électromagnétique (152) à un vaisseau (90) afin de produire un effet de soudage de vaisseau dans une section d'obturation spécifiée (96) du vaisseau (90) et/ou de couper le vaisseau (90) à l'intérieur de la section d'obturation spécifiée (96), dans lequel la ou les fibres optiques (110) comprennent au moins une région d'émission spécifiée (120A) qui est conçue pour émettre un rayonnement électromagnétique transféré à partir d'un coeur (116) au moyen d'un revêtement (115A) de la fibre optique (110) par la fibre optique (110) étant pliée à la ou aux régions d'émission spécifiées (120A) au-delà d'un seuil de pliage spécifié.

2. Forceps chirurgical selon la revendication 1, dans lequel au moins l'une des mâchoires (101) du forceps (92) comprend au moins une saillie (95A) agencée pour resserrer le vaisseau (90) avant l'actionnement de la ou des fibres optiques (110).

3. Forceps chirurgical selon la revendication 1 comprenant des éléments agencés pour concentrer l'énergie délivrée.

4. Forceps chirurgical selon la revendication 1, dans lequel l'embout d'obturation de vaisseau (100) comprend en outre au moins un élément de focalisation (111) agencé pour concentrer le rayonnement émis (152) dans un plan de coupe transversale de la ou des fibres optiques (110).

5. Forceps chirurgical selon la revendication 1, dans lequel au moins un élément optique comprenant la ou les fibres optiques (110) est conçu pour fonctionner dans un mode d'ablation pour réduire une épaisseur de la paroi du vaisseau avant le soudage et/ou la coupe, ou
dans lequel le forceps comprend des mâchoires (101), dans lequel au moins l'une des mâchoires (101) comprend au moins une saillie (95A) positionnée pour entrer en contact avec le tissu maintenu par l'embout d'obturation de vaisseau (100), ladite saillie comportant un élément d'abrasion ou d'ablation conçu pour réduire une épaisseur de la paroi du vaisseau ou couper le vaisseau.

6. Forceps chirurgical selon la revendication 1, dans lequel au moins l'une des mâchoires (101) du forceps (92) comprend au moins un élément expansible transversalement (102) agencé pour produire l'extension spécifiée de la section d'obturation spécifiée (96).

7. Forceps chirurgical selon la revendication 1, agencé en outre pour étendre mécaniquement la section d'obturation spécifiée (96) lors du soudage.

8. Forceps chirurgical selon la revendication 1, dans lequel l'embout d'obturation de vaisseau est construit à partir de matériaux non métalliques.

9. Forceps chirurgical selon la revendication 2, dans lequel la ou les fibres optiques (110) sont placées à l'intérieur de la ou des saillies (95 A).

10. Forceps chirurgical selon la revendication 4, dans lequel la focalisation du rayonnement émis a lieu dans un plan de coupe transversale de la ou des fibres optiques (110).

11. Forceps chirurgical selon la revendication 4, comprenant en outre au moins deux éléments de focalisation (111) positionnés et agencés *sur chacune des* mâchoires (101) afin de produire une extension de la section d'obturation spécifiée (96).
